# EUROPÄISCHE PATENTSCHRIFT

(11) **EP 0 381 174 B1**
(45) Veröffentlichungstag und Bekanntmachung des Hinweises auf die Patenterteilung: **24.08.1994**
(21) Anmeldenummer: 90101861.4
(22) Anmeldetag: 31.01.1990
(51) Int. Cl.: A61K 9/16, A61K 9/50, B01J 2/16

(54) **Verfahren zur Herstellung fester pharmazeutischer Zubereitungen**
Process for the manufacture of solid pharmaceutical preparations
Procédé de fabrication de préparations pharmaceutiques solides

(30) Priorität: 03.02.1989 DE 3903217
(43) Veröffentlichungstag der Anmeldung: 08.08.1990
(73) Patentinhaber: BOEHRINGER INGELHEIM VETMEDICA GMBH, 55216 Ingelheim (DE)
(72) Erfinder: Schleicher, Werner, Dr., D-6530 Bingen/Rhein (DE); Werner, Herbert, D-6507 Ingelheim am Rhein (DE)

(56) Entgegenhaltungen:
- EP-A- 0 281 204
- DE-A- 2 165 430

## Beschreibung

Die Erfindung betrifft ein Verfahren zur Herstellung fester pharmazeutischer Zubereitungen, wie z.B. von Granulaten, Pulvern, Pellets und kristalliner Wirkstoffe, die gegebenenfalls beschichtet sein können.

Von den in der pharmazeutischen Industrie verwendeten Verfahren ist die Herstellung derartiger fester Zubereitungen als endgültige Handelsform (z.B. Essgranulate, Trockensaftgranulate) sowie als Vorstufen zur Herstellung von Tabletten eine der wichtigsten Technologien.

Das dabei vom Grundprinzip her am häufigsten angewandte Verfahren bedient sich des Wirbelschichtgranulators, wobei alle einzelnen Herstellungsschritte (Mischen, Befeuchten, Granulieren, Trocknen und Endmischen) in einem Gerät durchgeführt werden können. Trotz dieses Vorteils konnte dieses Herstellungsverfahren in vielen Fällen keine befriedigenden Resultate liefern.

Neben Problemen, die mit der jeweiligen Rezeptur bzw. mit den eingesetzten Stoffen verbunden sind, ergeben sich wesentliche Nachteile, namentlich breite Kornverteilungsspektren und hohe Staub- bzw. Feinstaubanteile.

Diese Nachteile erschweren insbesondere die Reproduzierbarkeit der Qualität und die Validierung.

Des weiteren erfordern bestimmte Arzneimittelzubereitungen besondere Verfahrensweisen (z.B. eine möglichst vollständige Beschichtung - coating - der einzelnen Granulatkörner), um eine verbesserte Stabilität beispielsweise gegenüber Hitze- und Feuchtigkeitseinwirkung sowie gegenüber mechanischer Beanspruchung (Mischen, pneumatische Förderung, Verpressung) zu erlangen, damit auch nach längerer Lagerung unter ungünstigen Bedingungen die pharmakologische Aktivität des Arzneimittels erhalten bleibt.

Neben dem sog. Coating bereitet u.a. auch die aufbauende Granulation - Agglomeration - mit den bislang bekannten Verfahren immer wieder Schwierigkeiten.

Es ist die Aufgabe der vorliegenden Erfindung, ein Verfahren zur Verfügung zu stellen, das die Herstellung fester pharmazeutischer Zubereitungen - insbesondere für den humanpharmakologischen Bereich - mit engem Kornverteilungsspektrum und geringem Staubanteil auf einfache Weise ermöglicht.

Erfindungsgemäß wird diese Aufgabe durch das sogenannte Spaltkreisel-Dynamikfilter-Granulierverfahren (SKD-Granulierverfahren) gelöst. Bei dem SKD-Verfahren wird das pulverförmige Ausgangsmaterial in einem Spaltkreisel-Wirbelschicht-Granulierer (z.B. HSP-5/10, 250 oder 750 Fa. Hüttlin, Steinen) vorgelegt und mit einem geeigneten Überzugsmaterial vollständig überzogen. Die technische Apparatur ist in der deutschen Patentschrift 29 32 803 offenbart. - Die Europäische Patentschrift 146 680 offenbart eine dafür verwendbare Filteranordnung.

Nach diesem Verfahren können überzogene wirkstoffhaltige Teilchen hergestellt werden, die als Ausgangsmaterial für die Herstellung üblicher pharmazeutischer Zubereitungen Verwendung finden, so z.B. zur Herstellung von Tabletten, Kapseln u.a., oder aber auch direkt als pharmazeutische Zubereitung selbst verwendet werden können. Die überzogenen Teilchen können in Form von Pulvern, Granulaten oder Pellets je nach eingesetztem Ausgangsmaterial erhalten werden. Je nach eingesetztem Überzugsmaterial können die erhaltenen Überzüge eine die Wirkstofffreisetzung steuernde Funktion aufweisen oder aber auch lediglich geschmacksbildende oder schützende Funktionen aufweisen.

Das erfindungsgemäße Verfahren kann zur aufbauenden Granulation eingesetzt werden. Bei diesem Verfahren wird ein Trägermaterial vorgelegt, z.B. kleine Zuckerkügelchen und mit einer Wirkstofflösung besprüht.

Überraschenderweise wurde gefunden, daß unter Anwendung der in den zitierten Patentschriften beschriebenen Apparatur feste pharmazeutische Zubereitungen - z.B. auch in Form eines wirkstoffhaltigen Trägermaterials - hergestellt werden können, die eine engere Korngrößenverteilung und einen geringeren Staubanteil aufweisen als entsprechende Präparate, die in einem der herkömmlichen Wirbelschichtgranulierer hergestellt und überzogen wurden. Weitere Vorteile gegenüber den auf herkömmliche Weise hergestellten Präparaten sind, daß das nach dem erfindungsgemäßen Verfahren hergestellte Präparat eine sehr enge Korngrößenverteilung aufweist, die in weiten Bereichen einstellbar ist, daß das Präparat eine bessere Rieselfähigkeit aufweist und daß der Geruch intensiv riechender Ausgangsprodukte stark zurückgedrängt wird.

Zum Einsatz in dem erfindungsgemäßen Verfahren eignen sich sowohl feste als auch flüssige Wirkstoffe. Flüssige pharmazeutische Wirkstoffe können in der Weise verarbeitet werden, daß diese auf ein geeignetes vorgelegtes Trägermaterial aufgesprüht und anschließend gegebenenfalls mit einem Überzug beschichtet werden.

Als Wirkstoffe eignen sich unter anderem pharmazeutische Wirkstoffe insbesondere solche Wirkstoffe, die in Human-Arzneimitteln Verwendung finden - wie z.B. Ranitidin, Cimetidin, Atenolol, Enalapril, Captopril, Nifedipin, Naproxen, Diclofenac, Diclofenac-Natrium, Piroxicam, Cefaclor, Diltiazem, Ketotifen, Ketofifen-hydrogenfumarat, Salbutamol, Propranolol, Amoxicillin, Triamteren, Norethisteron, Mestranol, Cefotoxamin, Cefotoxamin-Natrium, Ceftriaxon, Ceftriaxon-Dinatrium, Cefalexin, Dipyridamol, Alprazolam, Cefoxitin, Ciclosporin, Metoprololtartrat, Aciclovir, Sulindac, Clavulansäure, Methyldopa, Nicardipin, Pentoxifyllin, Glyceroltrinitrat, Timolol, Idebenon, Terfenadin, Tamoxifendihydrogencitrat, Prazosin, Doxorubicin, Amilorid, Amilorid HCl, Hydrochlorothiazid, Dihydroergocornin, Dihydroergocorninmethansulfonat, Erythromycin, Erythromycinstearat, Triazolam, Latamoxef, Cromoglicinsäure, Ceftazidim, Clenbuterol, Bromhexin Oxytetracyclin, Dexamethason-21-isonicotinat, Sulfadiazin, Cimaterol, Aditoprim, Mederantil, Climazolam, Carprofen, Coffein, Catapresan und Acetylsalicylsäure - oder alle pharmazeutisch bzw. lebensmittelrechtlich zugelassenen Vitamine - wie z.B. Vitamin A, A₁, A₂, B₁, B₂, B₄ B₆, B₁₂, C (Ascorbinsäure). Ascorbylpalmitat und weitere pharmakologisch verträgliche Derivate der Ascorbinsäure, D, D₁, D₂, D₃, D₄, E, H, K, K₁, K₂, P und Q - oder Wirkstoffe - wie z.B. Avoparcin, Flavopholipol, Monensin, Monensin-Natrium, Salinomycin, Carbadox, Nitrovin und Olaquindox, sowie Wirkstoffe, wie sie in der Roten Liste 1989 (Editio Cantor Verlag für Medizin und Naturwissenschaften GmbH und Co. KG, Aulendorf/Württemb.) aufgeführt sind auf die hiermit inhaltlich Bezug genommen wird.

Als Überzugsmaterialien sind Verbindungen geeignet, wie sie auch als Überzugsmaterialien in der pharmazeutischen Galenik verwendet werden, so z.B. Polyacrylate, Polysaccharide, anorganische Überzugsmaterialien, wie z.B. Silikate oder Carbonate, soweit sie in Wasser oder leichtflüchtigen organischen Lösungsmittel oder Lösungsmittelgemischen löslich oder gut suspendierbar sind. In Abhängigkeit vom jeweiligen Wirkstoff sind auch Fette, Lipide, Lecithin, Wachse und Tenside als Überzugsmaterialien geeignet. Als anorganische Überzugsmaterialien sind weiterhin die folgenden Verbindungen geeignet: Bentonit, Montmorillonit, Calciumsilikat, Kaolinit-Tone, Kieselgur, Kieselsäuren (gefällt und getrocknet), Natriumaluminiumsilikat, Siliciumdioxid, Perlit, Vermiculit. Als Überzugsmaterialien mit basischem Charakter können beispielsweise Calciumhydrogenorthophosphat, Calciumoxid, Calciumtetrahydroorthophosphat, Diammoniumhydrogenorthophosphat, Dicalciumdiphosphat, Dinatriumdihydrogendiphosphat, Dinatriumhydrogenorthophosphat, Kalimdihydrogenorthophosphat und/oder Natriumdihydrogenorthophosphat verwendet werden. Weitere Überzugsmaterialien sind Cellulosen, insbesondere Hydroxypropylmethylcellulose, Natrium-carboxymethylcellulose, Hydroxyethylcellulose und Methylcellulose. Weiterhin geeignet sind Ethylcellulose, Celluloseacetatphthalat, Celluloseacetatsuccinat, Hydroxypropylmethylcellulosephthalat, Polyvinylacetat, Polyvinylpyrrolidone, Alginsäuren, Polyethylenglycole, Hexadecylalkohole, Hydroxypropylcellulose. Das Überzugsmaterial kann auch aus einer Mischung der aufgeführten Überzugsmaterialien zusammengesetzt sein.

Als Überzugsmaterialien sind ferner auch an sich bekannte Verbindungen geeignet, die eine verzögerte Freisetzung des Wirkstoffes bewirken. Hierzu gehören beispielsweise Überzüge aus Polyacrylaten, (Eudragit E 30 D, E 100 u.a. der Fa. Röhm, Darmstadt) und Polyethylenglycolen.

Als Trägerstoffe eignen sich z.B. Lactose, Saccharose, Glucose, Zucker, Maisstärke, Calciumcarbonat und auch alle als überzugsmaterialien genannten Stoffe.

Die minimal mögliche Größe der als Trägermaterial eingesetzten Partikel liegt bei ca. 0,5 »m - die Obergrenze bei ca. 1000 »m,. Der bevorzugte Größenbereich liegt zwischen 10 und 300 »m.

Als Lösungsmittel eignen sich alle niedrigsiedenden und pharmazeutisch unbedenklichen Lösungsmittel bzw. Lösungsmittelgemische, die sich gegenüber den Träger- und Beschichtungsmaterialien sowie gegenüber den Wirkstoffen inert verhalten. Bevorzugtes Lösungsmittel ist Wasser.

Der Anteil des Überzugsmaterials beträgt zwischen 2,5 und 30 Gew.%, bevorzugt 4 bis 20 Gew. %, besonders bevorzugt sind 5 bis 7 Gew. % bezogen auf das eingesetzte Ausgangsmaterial einschließlich eventuellen Trägermaterials. Es ist hierbei selbstverständlich, daß auch höhere Anteile an Überzugsmaterial eingesetzt werden können, wobei im Rahmen der Erfindung angestrebt wird, die Menge an Überzugsmaterial möglichst gering zu halten. Das erfindungsgemäße Verfahren läuft wie folgt ab:
Das pulverförmige Ausgangsmaterial - beispielsweise mit einer Partikelgröße zwischen 0,5 und 60»m - wird in der zuvor genannten Spaltkreisel-Dynamikfilter-Granulier-Apparatur vorgelegt. Die über die rotierenden Spaltkreisel-Spalten in das Pulverbett einströmende Luft fluidisiert das Produkt. Anschließend wird in diese Fluidisierungszone eine ca. 3 bis 10 %ige, bevorzugt 5 bis 7 %ige Lösung bzw. Suspension des Überzugsmaterials, die als Granulierungs- bzw. Coatingflüssigkeit dient, von unten in die Apparatur eingesprüht. Die einzelnen Prozeßparameter, wie z.B. Zuluftmenge, Zulufttemperatur, Ablufttemperatur, Abluftfeuchte, Sprühdüsendurchmesser, Sprührate, Drehzahl Spalt/Düse sind u.a. von der Größe der verwendeten Apparatur abhängig. Konkrete Angaben hierzu finden sich in den Beispielen. Wenn es gewünscht wird, kann der Prozess unter Anwendung eines Inertgases, wie z.B. Stickstoff gefahren werden.

Gegenüber herkömmlichen Granulierverfahren, z.B. der Wirbelschichtgranulierung, ist die gesamte Prozeßzeit stark verkürzt. Eine nachfolgende Siebung zur Beseitigung von Sekundäragglomeraten ist nicht erforderlich. Ein weiterer Vorteil ist, daß das Präparat nicht nur aufbauend granuliert wird, sondern der Überzug das Ausgangsmaterial vollständig als Coating umhüllt. Der Unterschied zu herkömmlichen Granulierverfahren, bei denen der Überzug unvollständig ist, kann durch Aufnahmen mit dem Rasterelektronenmikroskop belegt werden.

Aufgrund der sehr glatten Oberfläche, zeigt das erfindungsgemäße Produkt eine sehr gute Rieselfähigkeit. Das nach dem erfindungsgemäßen Verfahren hergestellte Granulat zeigt eine typische, relativ enge Korngrößenverteilung. In Abbildung I ist die Korngrößenverteilung am Beispiel des Clenbuterols dargestellt. Bei der herkömmlichen Wirbelschichtgranulation kann ein solches Spektrum nur nach mehrfacher Siebung und Mischung erzielt werden.

Im Gegensatz zu herkömmlich hergestellten Produkten zeigt das erfindungsgemäße praktisch keinen Feinstaubanteil und keinen Abrieb. Die mit Hilfe des SKD-Verfahrens hergestellten Arzneimittelzubereitungen zeigen im Stauber-Heubach-Test (Fresenius; Z. Anal. Chem. 318 (1984) 522-524) keinen nachweisbaren Abrieb von Wirksubstanz. Damit ist ausgeschlossen, daß es beim bestimmungsgemäßen Umgang mit den nach dem erfindungsgemäßen Verfahren hergestellten Zubereitungen zu einer Gesundheitsgefährdung durch wirkstoffhaltige Feinstäube kommt.

Ein weiterer Vorteil des erfindungsgemäß hergestellten Granulats besteht in der Verhinderung von Staubexplosionen, die beim Umgang mit feinpulverigen Substanzen oft ein Risiko darstellen.

Die aufgezählten Eigenschaften bieten wesentliche Vorteile bei der Weiterverarbeitung von Arneimittelzubereitungen, so z.B. beim Vermischen mit anderen Stoffen und bei der Weiterverarbeitung zu Tabletten und beim Befüllen von Kapseln.

Ein weiterer Vorteil wird durch die sehr homogene Wirkstoffverteilung bei den erfindungsgemäß hergestellten Produkten verkörpert, wodurch eine hohe Dosierungsgenauigkeit gewährleistet wird.

Ein weiterer Vorteil resultiert aus der sehr guten Rieselfähigkeit der erfindungsgemäß hergestellten Produkte, wodurch ein problemloses Abfüllen ermöglicht wird.

Ferner ist nicht mit Entmischungen beim Transport und der Weiterverarbeitung zu rechnen.

Ein weiterer Vorteil des erfindungsgemäßen Verfahrens besteht darin, daß - im Gegensatz zu den Wirbelschichtgranulationsverfahren - auch mikronisierte Ausgangsmaterialien mit Korngrößen bis zu 0,5 »m eingesetzt werden können, wobei die erzielten Ausbeuten unter Zuhilfenahme eines Feinstaubrecycling-Systems nahezu 100 % erreichen können.

Ferner können durch das erfindungsgemäße Verfahren Trägermaterialien mit einem vorbestimmten Korngrößenspektrum erhalten werden.

Des weiteren eignet sich das erfindungsgemäße Verfahren zur Geschmacksmaskierung.

### Beispiel 1

### Zusammensetzung

| Bestandteile: | g/100 g | g/10 kg |
|---|---|---|
| (01) Clenbuterol | 0,0016 | 0,160 |
| (02) Mannit | 0,3184 | 31,840 |
| (03) Lactose fein (D 80) | 60,000 | 6.000,000 |
| (04) Maisstärke, getrocknet* | 33,680 | 3.368,000 |
| (05) Kollidon 25 (PVP**) | 1,000 | 100,000 |
| (06) Stärke, löslich | 5,000 | 500,000 |
| | 100,000 | 10.000,000 |

| | | |
|---|---|---|
| * Wird Maisstärke ungetrocknet eingesetzt, so muß ein Gewichtszuschlag von 7 % eingerechnet werden | | |
| ** PVP = Polyvinylpyrrolidon | | |

### Vorlage im SKD

6.000 g Lactose, grob (Spezifikation D 20)
3.368 g Maisstärke, getrocknet

### Herstellung der Granulierlösung für einen 10 kg-Ansatz:

### Lösung 1:

0,160 g Clenbuterol werden in 50 ml H₂O gelöst.

### Lösung 2:

In ein Thermostatgefäß werden 800 ml H₂O bei Raumtemperatur vorgelegt und 500 g lösliche Stärke unter Rühren suspendiert.

In diese Suspension wird die Lösung 1 eingerührt.

### Lösung 3:

In ein 5l-Becherglas werden 2.500 ml H₂O vorgelegt. 31,84 g Mannit werden unter Rühren gelöst und die Lösung bis zum Sieden erhitzt.

### Lösung 4:

Die heiße Mannitlösung wird unter Rühren zu Lösung 2 zugegeben, wobei die lösliche Stärke sofort sichtbar verkleistert (Aufquellung).

### Lösung 5:

650 ml H₂O werden im Becherglas bis zum Sieden erhitzt und 100 g Kollidon 25 unter Rühren darin gelöst.

### Granulierlösung:

Lösung 5 wird jetzt in Lösung 4 (in Thermostatgefäß) eingerührt.

Die zum Versprühen erforderliche Temperatur von 50°C wird eingestellt.

### Granulierung im SKD*:

- Herstellung:: Der SKD wird auf ca. 60°C vorgeheizt. Dann werden die vorher bereitgestellten Füllstoffe, ungesiebt, vorgelegt und ca. 2-3 min. gemischt (ca. 50 Nm³N₂/h). Inzwischen wird der SKD weiter aufgeheizt
(Temperatureinstellung für die Zuluft 100°C).
Nach dem Mischen wird die Granulierlösung bei einer Temperatur von ca. 50°C aufgesprüht.
Nachdem die Granulierlösung komplett eingesprüht ist, wird das Thermostatgefäß mit zweimal 20 ml H₂O ausgespült, die ebenfalls eingesprüht werden.
Das Granulat wird bis zu einer Produkttemperatur von 60°C getrocknet.
(Die Restfeuchte liegt dann bei ca. 4,5-6 % nach der Methode von Karl Fischer)

*SKD = Spaltkreisel-Dynamikfilter-Granuliergerät

### Beispiel 2

- Zusammensetzung:: 95,0 g Wirkstoff
5,0 g Methylcellulose
1̅0̅0̅,̅0̅ g̅ W̅i̅r̅k̅s̅t̅o̅f̅f̅g̅r̅a̅n̅u̅l̅a̅t̅
- Methode:: Das pulverförmige Ausgangsmaterial Wirkstoff mit einer Partikelgröße von 1 - 40 »m (Hauptanteil: 10 - 15 »m) wird im SKD vorgelegt. Es wird eine 5%ige Methylcelluloselösung hergestellt, die als Granulierflüssigkeit dient. Die über die rotierenden Spaltkreisel-Spalten in das Pulverbett einströmende Luft fluidisiert das Produkt und in diese Fluidisierungszonen wird jetzt die Granulierflüssigkeit von unten eingesprüht.
- Prozeßparameter:: Zuluftmenge: 150 - 250 m³/h
Zulufttemperatur: 60 - 70°C
Sprühdüsendurchmesser: 1,2 mm
mittlere Sprührate: 18 ml/min
Drehzahl Spalt/Düse: 3 Upm
Die gesamte Sprühzeit beträgt ca 155 min, die
Trocknungsphase ca. 25 min.
Die Gesamtprozeßzeit beträgt demnach ca. 180 min.
Dem gegenüber beträgt die Gesamtprozeßzeit für ein im konventionellen Wirbelschichtgranulator /z.B. WSG 200 der Fa. Glatt) hergestelltes Wirkstoff-Granulat mehr als 8h, wobei aufgrund von großen Sekundäragglomeraten und breitem Kornverteilungsspektrum noch eine zusätzliche Siebung und Mischung erforderlich ist.
Nach bereits 15 min ist eine Granulatbildung erkennbar. Die Granulierphase geht, vermutlich stufenlos, in eine Coatingphase über, bei der das primär gebildete Granulat allmählich von Methylcellulose eingehüllt wird. Aufnahmen mit dem Rasterelektronenmikroskop 600/1800 fache Vergrößerung zeigen unterschiedliche glatte Oberflächen bei 2 Mustern, die mit 5,14 bzw. 9,48% Methylcellulose beschichtet wurden, wobei die höhere Coatingkonzentration zu einer glatteren Oberfläche führt.
Das mittels SKD hergestellte Endprodukt hat praktisch keinen Feinstaubanteil mehr (Partikel kleiner 45 »m = 0%) und weist ein enges Kornverteilungsspektrum auf (Partikel 200 - 400 »m = 92%). Damit verbunden ist ein gutes Fließverhalten des Granulats (Böschungswinkel: 33,6°, Düsenöffnung 6 mm, Fließzeit für 200 ml: ca. 75 sec.).

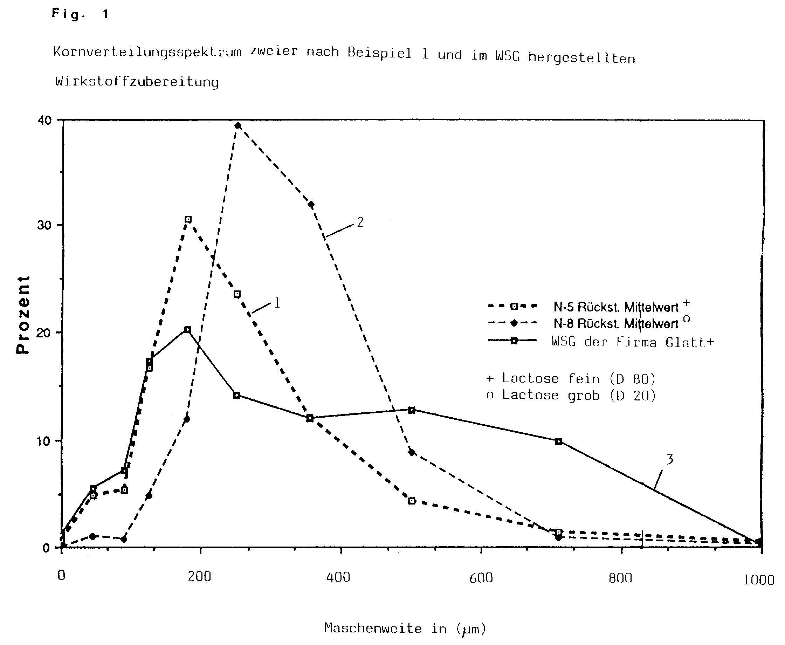

## Patentansprüche

1. Verfahren zur Herstellung fester pharmazeutischer Zubereitung mit enger Korngrößenverteilung, dadurch gekennzeichnet, daß die pharmazeutische Zubereitung durch aufbauende Granulation unter Anwendung des Spaltkreisel-Dynamikfilter-Granulierverfahrens hergestellt wird, wobei ein Überzug das Ausgangsmaterial vollständig als Coating umhüllt.

2. Verfahren nach Anspruch 1, dadurch gekennzeichnet, daß das Überzugsmaterial aus einem physiologisch unbedenklichen Polymer besteht.

3. Verfahren nach Anspruch 1, dadurch gekennzeichnet, daß das Überzugsmaterial aus einem physiologisch unbedenklichen anorganischen Hilfsstoff besteht.

4. Verfahren zur Herstellung wirkstoffhaltiger Teilchen in Form von Pulvern, Granulaten oder Pellets, dadurch gekennzeichnet, daß die Trägerteilchen unter Anwendung des Spaltkreisel-Dynamikfilter-Granulierverfahrens mit einer wirkstoffhaltigen Lösung oder Suspension besprüht werden.

5. Verfahren zur Herstellung überzogener wirkstoffhaltiger Teilchen nach Anspruch 4, dadurch gekennzeichnet, daß die wirkstoffhaltigen Teilchen unter Anwendung des Spaltkreisel-Dynamikfilter-Granulierverfahrens mit einer einen Überzug bildenden Lösung besprüht werden.

## Claims

1. Process for producing a solid pharmaceutical preparation with a narrow particle size distribution, characterised in that the pharmaceutical preparation is produced by build-up granulation using the slotted gyrodynamic filter granulation method, a coating completely covering the starting material.

2. Process according to claim 1, characterised in that the coating material consists of a physiologically acceptable polymer.

3. Process according to claim 1, characterised in that the coating material consists of a physiologically acceptable inorganic excipient.

4. Process for preparing particles which contain active substance, in the form of powders, granules or pellets, characterised in that the carrier particles are sprayed with a solution or suspension which contains the active substance using the slotted gyrodynamic filter granulation method.

5. Process for preparing coated particles containing active substance according to claim 4, characterised in that the particles containing active substance are sprayed with a solution which forms a coating using the slotted gyrodynamic filter granulation method.

## Revendications

1. Procédé de fabrication d'une préparation pharmaceutique solide à répartition granulométrique étroite, caractérisé en ce que la préparation pharmaceutique est fabriquée par granulation d'élaboration au moyen du procédé de granulation à filtre dynamique et à rotor à fentes, un revêtement entourant totalement le produit de départ sous forme d'un enrobage.

2. Procédé selon la revendication 1, caractérisé en ce que le matériau de revêtement consiste en un polymère irréprochable du point de vue physiologique.

3. Procédé selon la revendication 1, caractérisé en ce que le matériau de revêtement consiste en un adjuvant inorganique irréprochable du point de vue physiologique.

4. Procédé de fabrication de particules contenant un principe actif sous forme de poudres, de granulés ou de pastilles, caractérisé en ce que les particules de support sont aspergées d'une solution ou suspension contenant un principe actif au moyen du procédé de granulation à filtre dynamique et à rotor à fentes

5. Procédé de fabrication de particules revêtues contenant un principe actif selon la revendication 4, caractérisé en ce que les particules contenant un principe actif sont aspergées d'une solution formant un revêtement au moyen du procédé de granulation à filtre dynamique et à rotor à fentes.
